# EUROPEAN PATENT SPECIFICATION

(11) **EP 0 533 936 B1**
(45) Date of publication and mention of the grant of the patent: **19.04.1995**
(21) Application number: 92901905.7
(22) Date of filing: 27.12.1991
(51) Int. Cl.: C07D 307/32, A61K 7/46

(54) **PROCESS FOR PRODUCING CIS-WHISKEY-LACTONE**
VERFAHREN ZUR HERSTELLUNG VON CIS-WHISKEY-LACTON
PROCEDE POUR PRODUIRE UNE CIS-LACTONE DE WHISKY

(30) Priority: 27.12.1990 JP 407648/90
(43) Date of publication of application: 31.03.1993
(73) Proprietor: Japan Tobacco Inc., Shinagawa-ku, Tokyo 140 (JP); YUKI GOSEI KOGYO CO., LTD., Chiyoda-ku Tokyo 102 (JP)
(72) Inventor: EBATA, Takashi, Japan Tobacco Inc., Life Science, Yokohama-shi, Kanagawa-ken 227 (JP); MATSUMOTO,Katsuya, Japan Tobacco Inc.Life Science, Yokohama-shi, Kanagawa-ken 227 (JP); KOSEKI, Koshi, Japan Tobacco Inc., Life Science, Yokohama-shi, Kanagawa-ken 227 (JP); KAWAKAMI, Hiroshi, Japan Tobacco Inc Life Science, Yokohama-shi, Kanagawa-ken 227 (JP); MATSUSHITA,Hajime, Japan Tobacco Inc Life Science, Yokohama-shi, Kanagawa-ken 227 (JP); YOSHIKOSHI, Hajime, Yuki Gosei Kogyo Co., Ltd., Itabashi-ku, Tokyo 174 (JP); OKANIWA, Masakazu, Yuki Gosei Kogyo Co., Ltd., Itabashi-ku, Tokyo 174 (JP)
(74) Representative: Reinhard - Skuhra - Weise & Partner
(86) International application number: PCT/JP91/01780
(87) International publication number: WO 92/12143

(56) References cited:
- JP-A-63 041 473
- LIEBIGS ANNALEN DER CHEMIE no. 12, 1986, WEINHEIM DE pages 2112 - 2122 C. G]NTHER ET AL. 'Stereoisomere Aromastoffe, XII. 3-Methyl-4-octanolid "Quercuslacton, Whiskylacton" - Struktur und Eigenschaften der Stereoisomeren'
- TETRAHEDRON, (INCL. TETRAHEDRON REPORTS) vol. 45, no. 10, 1989, OXFORD GB pages 3151 - 3162 J. SALLAUN ET AL. 'OPTICALLY ACTIVE 2-VINYLCYCLOBUTANONES'
- DATABASE WPIL Week 8818, Derwent Publications Ltd., London, GB; AN 88-123680

## Description

### [Technical Field]

The present invention relates to a method of producing natural type cis-whiskey lactone contained in whiskey, wine and the other.

### [Prior Art]

Whiskey lactone (3-methyl-4-octanolide) is one of perfume components of whiskey and wine.

There are stereoisomers in the natural whiskey lactone, which are of the trans-type or cis-type in accordance with the configuration of methyl group at 3-position thereof and butyl group at 4-position thereof.
As compared to trans-whiskey lactone
[(3S,4R)-3-methyl-4-octanolide] (D), generally, a less amount of cis-whiskey lactone
[(3S,4S)-3-methyl-4-octanolide] (A) is contained, for example, in whiskey and wine. However, cis-whiskey lactone (A) is superior in the characteristic of perfume. 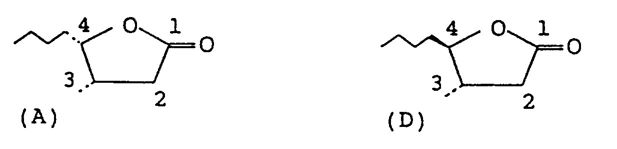
However, means for selectively synthesizing natural type cis-whiskey lactone (A) having superior properties than those of trans-type isomer (D) as described above has not been known.

Liebigs Annalen der Chemie, No. 12, 1986, pages 2112 - 2122, disclose a method, how all four stereoisomers of 3-methyl-4-octanolide can be produced. It is taught to separate a racemic cis/trans octanolide into the two cis- and the two trans-isomers chromatographically. Furthermore, it is disclosed how to hydrolyse a lactone ring with potassium hydroxide and to protect the carboxy group subsequently yielding the isopropyl carboxylic ester. Diastereomeric pairs of the acid-protected gamma-hydroxyacid are formed by reaction with an optically active carboxylic acid, and are separated from each other by liquid chromatography. It is disclosed how to perform the hydrolysis of the diastereomers and the relactonization to obain the four stereoisomers of whiskey-lactone. No reaction at the 4-hydroxy group is suggested to invert the configuration of the chiral centre.

Synthesis, No. 1, 1981, pages 1-28, discloses the use of diethyl azodicarboxylate and triphenylphosphine in synthesis and transformation of natural products. However, there is no indication for a combination of these documents. Furthermore, it is well-known that the Mitsunobu reaction proceeds according to Sn2 reaction mechanism. In the case of 3,4-trans compound (III) which is a substrate of the method of the present invention, the steric hindrance to the 4-methyl group is large because of the influence from the methyl group at a 3-position. Therefore, it cannot be expected that the Mitsunobu reaction proceeds to compound (II) of the present invention.

### (Disclosure of the Invention]

It is the object of the present invention to provide a method of producing cis-whiskey lactone which is able to selectively and easily synthesize natural type cis-whiskey lactone.

In order to solve the above problem, the inventor carefully reviewed and paid attention to the fact that the trans-whiskey lactone and cis-whiskey lactone differ only in the absolute configuration of a butyl group at 4-position thereof, found out that the trans-whiskey lactone can be converted to the cis-whiskey lactone by reversing the solid configuration of the butyl group at 4-position, and completed the present invention.

The present invention is:
Method of producing cis-whiskey lactone, having,
(a) step of hydrolyzing a lactone portion of 3,4-trans-whiskey lactone [(3S,4R)-3-methyl-4-octanolide] indicated by general formula (D) and adding an alkyl group to the hydrolyzed portion, thereby obtaining 3,4-trans compound represented by general formula (C),
(b) step of making 3,4-trans compound (C) obtained in step (a) react with acyl compound in the presence of triphenylphosphine and azodicarboxylate, thereby obtaining 3,4-cis compound (B) indicated by general formula (B), and
(c) step of hydrolyzing 3,4-cis compound (B) obtained in step (b) and lactonizing the hydrolyzed compound, thereby obtaining 3,4-cis-whiskey lactone [(3S,4S)-3-methyl-4-octanolide] (A) indicated by general formula (A) 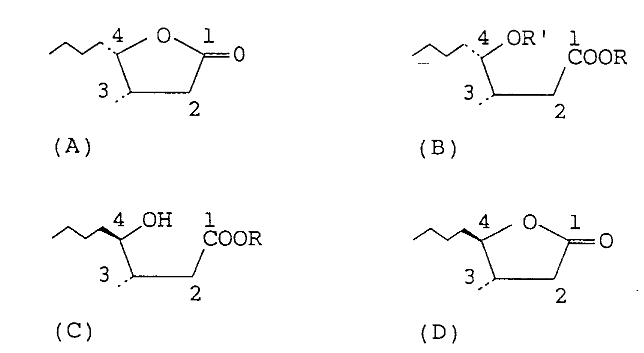 (wherein R represents a general alkyl group and R' represents a general acyl group).

The method of producing cis-whiskey lactone of the present invention will be described below in detail.

Trans-whiskey lactone (D) used as a starting substance in the present invention can be produced, for example, by a well-known producing method described in "Heterocycles No. 31, pp. 1585-1588 (1990)".

Hydrolysis of the lactone portion in step (a) can be treated, for example, by a solution of an alkali such as sodium hydroxide, and potassium hydroxide. As a result, a lactone ring of the trans-whiskey lactone is broken between a carbon at 1-position of the lactone and oxygen and trans compound (D₂) as indicated in the following formula can be obtained. 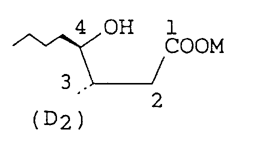
(In the formula, M represents an alkali metal.)

Then, trans compound (D₂) is made to react with, for example, alkyl halide such as isopropyl bromide, butyl bromide in an appropriate solvent, thereby adding alkyl group to a carbonyl group at 1-posiiton of the compound (D₂) and obtaining trans compound (C). The appropriate solvent to be used in this step is, for example, an organic solvent such as dimethyl formamide, dimethyl sulfoxide, but it is not particularly limited.

In the reverse reaction, i.e. Mitunobu reaction of the buthyl group at 4-position of trans compound (C) in the step (b), trans compound (C) obtained in the step (a) is made to react with, for example, acetic acid, benzoic acid, or, acyl compound of their derivative in the presence of triphenylphosphine and azodicarboxylate, in an appropriate solvent, thereby reversing the configuration of butyl group at 4-position of trans compound (C). As a result, cis compound (B) in which butyl group of 4-position thereof and methyl group at 3-position thereof are mutually configurated in the cis-type can be obtained. The appropriate solvent to be used in this step is, for example, an organic solvent such as ether, tetrahydrofuran, toluene, but it is not particularly limited.

In the step (c), at first, the alkyl group added to 1-position carboxyl group of the cis compound (B) and acyl group at 5-position can be eliminated, for example, by hydrolyzing the cis compound (B) in the basic condition.

A basic compound to be used for the elimination reaction in the step (c) is, for example, metallic oxide (potassium hydroxide, sodium hydroxide, lithium hydroxide, etc.), carbonate (potassium carbonate, sodium carbonate, etc.), or metal alkoxide (alkoxide sodium isopropoxide, potassium butoxide, etc.)

A solvent to be used in the step (c) is, for example, water, alcohol (methanol, ethanol, etc.), tetrahydrofuran, or these mixed solvent, but it is not particularly limited.

After the above elimination reaction, the lactone ring is closed by making the reactant solution acid, thereby obtaining cis-whiskey lactone (A). An acid substance to be used for this lactonization is, for example, mineral acids (hydrochloric acid, sulfuric acid, etc.), organic acids (paratoluenesulfonic acid, etc.)

In the above description, the case of converting (3S,4R)-3-methyl-4-octanolide(D) in the trans-type of the whiskey lactone to (3S,4S)-3-methyl-4-octanolide (A) in the cis-type was described. However, conversion of (3R,4S)-3-methyl-4-octanolide shown by the following general formula (D') to (3R,4R)-3-methyl-4-octanolide shown by general formula (A') can also be performed in a similar manner as the above reaction. 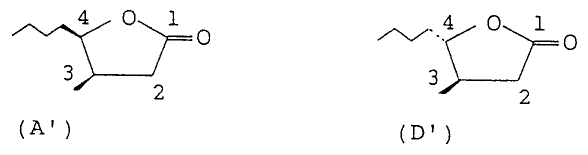
As described above, according to the method of producing cis-whiskey lactone of the present invention, effects, which conclude that the natural type cis-whiskey lactone with high utility can be selectively and easily synthesized, can be accomplished.

### [Most Preferable Manner of sample]

The present invention will be described in further detail in the following sample.

### Example 1

### Step A : Production of isopropyl (3S,4S)-4-hydroxy-3-methyloctanoate.

1.07g (6.86 mmol) of (3S,4R)-3-methyl-4-octanolide (trans-whiskey lactone) produced according to the producing method described in the above Heterocycles No. 31 was dissolved in a mixed solvent of methanol (7 ml) and water (1.4 ml). 491 mg (7.54 mmol) of potassium hydroxide under the ice cooling was added to the obtained solution. Further, this solution was stirred for 5 hours at room temperature and then concentrated. Ether was added to the obtained residue, the obtained solution was concentrated again. Further, after this operation was repeated once again, reduced-pressure drying was performed for 6 hours.

After this, the dried residue was dissolved in 10 ml of anhydrous dimethylformamide, 1.69g (13.7 mmol) of isopropyl bromide was added to the solution, and this solution was stirred for one night at room temperature. Then, after 30 ml of water was added to the reactant solution and extracted 5 times by ether. The solution which was obtained by combining each extracted solutions was washed by water and saturated saline water sequentially, dried by anhydrous sodium sulfate, and concentrated. In this way, 1.60g of crude product of isopropyl (3S,4S)-4-hydroxy-3-methyloctanoate was obtained. Physical data of the obtained crude product were:
IR (film): γmax 3500(br), 2962(s), 2934(s), 2876(s), 1729(s), 1715(s), 1460(m), 1377(s), 1270(s), 1178(s), 1108(s), 975(s), 899(m) (in cm⁻¹ units)
This crude product was used in the following step without being purified.

### Step B : Production of isopropyl (3S,4S)-4-(3',5'-dinitrobenzoyloxy)-3-methyloctanoate.

1.60g of the crude product obtained in the step A was dissolved in 12 ml of anhydrous tetrahydrofuran. While the solution was stirred and cooled with ice, 2.70g (10.3 mmol) of triphenylphosphine, 2.18g (10.3 mmol) of 3,5-dinitro benzoic acid, and 1.79g (10.3 mmol) of diethyl azodicarboxylate were added in this solution. After stirred for three days at room temperature, this solution was poured into an iced water and extracted by ether. After washed with water and saturated saline water sequentially, this extracted solution was dried by anhydrous magnesium sulfate. After concentrated, the obtained residue was purified by silica-gel column chromatography (n-hexane : ether = 10 : 1 - 5 : 1), thereby obtaining 2.15g (76.5%) of isopropyl (3S,4S)-4-(3',5'-dinitrobenzoyloxy)-3-methyl octanoate. Physical data given in ppm units of the obtained product were:
¹H-NMR(CDCl₃) : δ 0.90(3H, t, J = 6.9 Hz), 1.09(3H, d, J = 6.8 Hz), 1.21(3H, d, J = 6.2 Hz), 1.23(3H, d, J = 6.2 Hz), 1.26 - 1.40(4H, m), 1.62 - 1.85(2H, m), 2.13 - 2.25(1H, m), 2.35 - 2.50(2H, m), 5.01(1H, m), 5.23 - 5.30(1H, m), 9.11 - 9.18(2H, m), 9.21 - 9.25(1H, m).

### Step C: Production of (3S,4S)-3-methyl-4-octanolide (cis-whiskey lactone)

380 mg (0.93 mmol) of isopropyl (3S,4S)-4-(3^{'},5'-dinitrobenzoyloxy)-3-methyl octanoate obtained in the step B was dissolved in 5 ml of methanol. 2% sodium hydroxide was added to adjust this solution at pH14. After this solution was stirred at one night at room temperature, 1N hydrochloric acid was added to the reactant solution, and the solution was adjusted at pH1. Thereafter, the solution was stirred for one hour at room temperature. Then, after the resultant solution was extracted by ether, the extracted solution was dried by anhydrous magnesium sulfite, and concentrated. After purified by column chromatography (n-hexane : ether = 10 : 1 - 2 : 1), the obtained residue was distilled at the reduced pressure and 122 mg (84.1%) of (3S,4S)-3-methyl-4-octanolide (cis-whiskey lactone) to be required was obtained. Physical data of the obtained cis-whiskey lactone were:
Boiling Point : 124 - 126°C/(17 mmHg) 2266 Pa
¹H-NMR(CDCl₃) : δ 0.92(3H, t, J = 7.0 Hz), 1.02(3H, d, J = 6.9 Hz), 1.20 - 1.75(6H, m), 2.20(1H, dd, J = 3.8 and 16.8 Hz), 2.51 - 2.64(1H, m), 2.70(1H, dd, J = 7.8 and 16.8 Hz), 4.40 - 4.48(1H, m) (ppm units)

## Claims

1. Method of producing cis-whiskey lactone, comprising,
(a) step of hydrolyzing a lactone portion of 3,4-trans-whiskey lactone shown by general formula (IV) and adding an alkyl group to the hydrolyzed portion, thereby obtaining 3,4-trans compound shown by general formula (III), 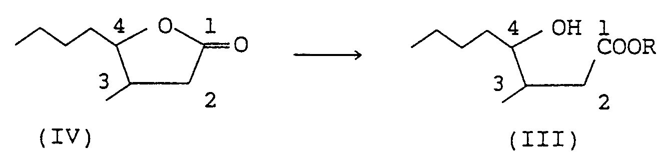 (wherein R represents a general alkyl group and R' a general acyl group)
(b) step of making 3,4-trans compound (III) obtained in the step (a) react with acyl compound in the presence of triphenylphosphine and azodicarboxylate, thereby obtaining 3,4-cis compound shown by general formula (II), and, 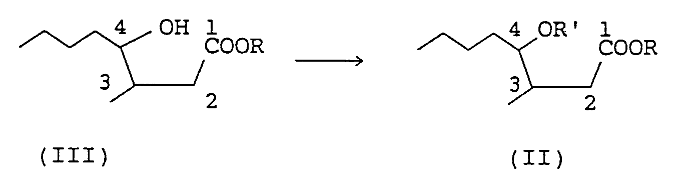 (wherein R and R' represent the same as the above)
(c) step of hydrolysing and lactonizing 3,4-cis compound (II) obtained in the step (b), thereby obtaining 3,4-cis-whiskey lactone shown by general formula (I) 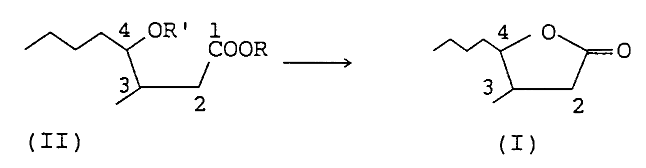 (wherein R and R' represent the same as the above).

2. Method of producing cis-whiskey lactone according to claim 1 comprising,
(a) step of hydrolyzing a lactone portion of (3S,4R)-3-methyl-4-octanolide shown by general formula (D) and adding an alkyl group to the hydrolyzed portion, thereby obtaining 3,4-trans compound shown by general formula (C), 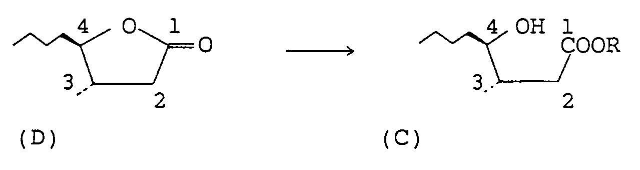 (wherein R represents a general alkyl group and R' a general acyl group)
(b) step of making 3,4-trans compound (C) obtained in the step (a) react with acyl compound in the presence of triphenylphosphine and azodicarboxylate, thereby obtaining 3,4-cis compound shown by general formula (B), and, 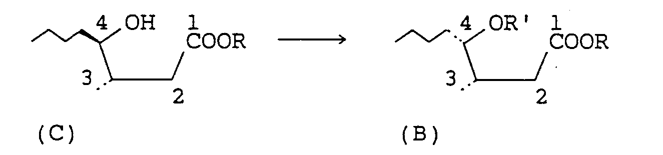 (wherein R and R' represent the same as the above)
(c) step of hydrolysing and lactonizing 3,4-cis compound (B) obtained in the step (b), thereby obtaining (3S,4S)-3-methyl-4-octanolide shown by general formula (A), 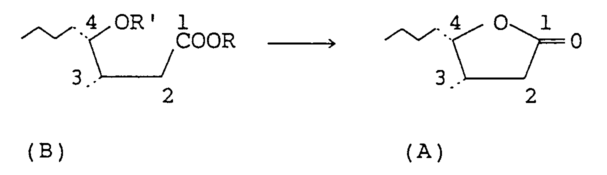 (wherein R and R' represent the same as the above).

3. Method of producing cis-whiskey lactone according to claim 1 comprising,
(a) step of hydrolysing a lactone portion of (3R,4S)-3-methyl-4-octanolide shown by general formula (D') and adding an alkyl group to the hydrolyzed portion, thereby, obtaining 3,4-trans compound shown by general formula (C'), 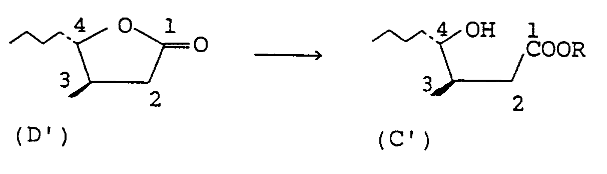 (wherein R represents a general alkyl group and R' a general acyl group)
(b) step of making 3,4-trans compound (C') obtained in the step (a) react with acyl compound in the presence of triphenylphosphine and azodicarboxylate, thereby obtaining 3,4-cis compound shown by general formula (B'), and 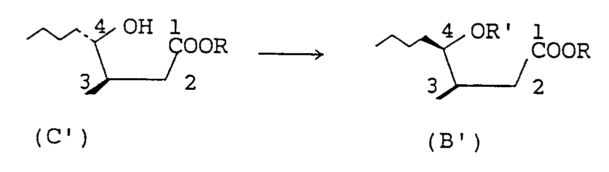 (wherein R and R' represent the same as the above)
(c) step of hydrolyzing and lactonizing 3,4-cis compound (B') obtained in the step (b), thereby obtaining (3R,4R)-3-methyl-4-octanolide indicated by general formula (A'), 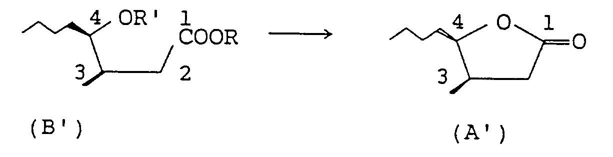 (wherein R and R' represent the same as the above).

## Patentansprüche

1. Verfahren zur Herstellung von cis-Whiskey-lacton, umfassend die nachfolgenden Schritte:
(a) Hydrolysieren des Lactonanteils von 3,4-trans-Whiskey-lacton mit der allgemeinen Formel (IV) und Anlagerung einer Alkylgruppe an den hydrolysierten Teil, wodurch die in der allgemeinen Formel (III) gezeigte 3,4-trans-Verbindung erhalten wird 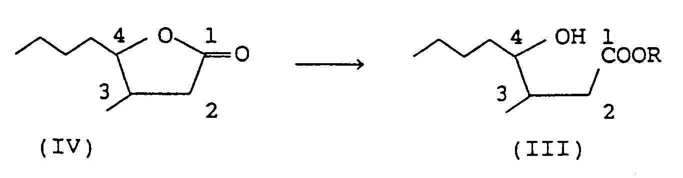 (wobei R eine allgemeine Alkylgruppe und R' eine allgemeine Acylgruppe repräsentieren),
(b) Umsetzen der im Schritt (a) erhaltenen 3,4-trans-Verbindung (III) mit einer Acylverbindung in Gegenwart von Triphenylphosphin und Azodicarboxylat, wodurch die in der allgemeinen Formel (II) gezeigte 3,4-cis-Verbindung erhalten wird 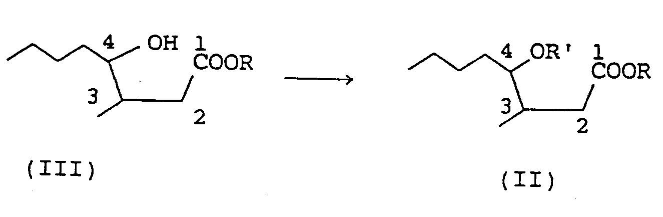 (worin R und R' die gleichen Bedeutungen wie oben angegeben aufweisen), und
(c) Hydrolysieren und Lactonisieren der im Schritt (b) erhaltenen 3,4-cis-Verbindung (II), wodurch das in der allgemeinen Formel (I) gezeigte 3,4-cis-Whiskey-lacton erhalten wird 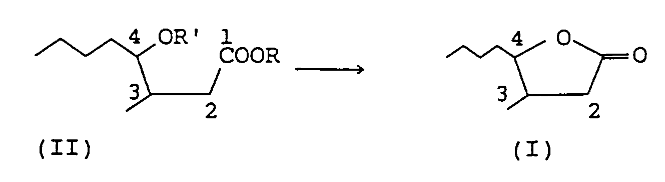 (worin R und R' die gleichen Bedeutungen wie oben angegeben aufweisen).

2. Verfahren zur Herstellung von cis-Whiskey-lacton nach Anspruch 1, umfassend die nachfolgenden Schritte:
(a) Hydrolysieren des Lactonanteils von (3S,4R)-3-Methyl-4-octanolid der allgemeinen Formel (D) und Anlagerung einer Alkylgruppe an den hydrolysierten Teil, wodurch die in der allgemeinen Formel (C) gezeigte 3,4-trans-Verbindung erhalten wird 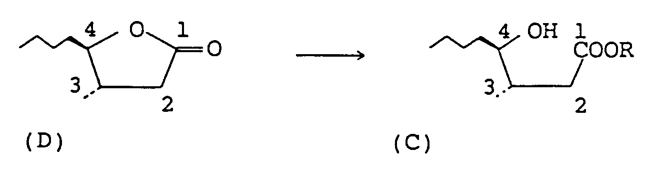 (wobei R eine allgemeine Alkylgruppe und R' eine allgemeine Acylgruppe repräsentieren),
(b) Umsetzen der im Schritt (a) erhaltenen 3,4-trans-Verbindung (C) mit einer Acylverbindung in Gegenwart von Triphenylphosphin und Azodicarboxylat, wodurch die in der allgemeinen Formel (B) gezeigte 3,4-cis-Verbindung erhalten wird 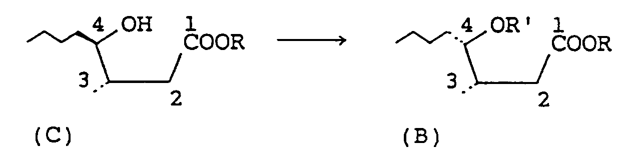 (worin R und R' die gleichen Bedeutungen wie oben angegeben aufweisen), und
(c) Hydrolysieren und Lactonisieren der im Schritt (b) erhaltenen 3,4-cis-Verbindung (B), wodurch das in der allgemeinen Formel (A) gezeigte (3S,4S)-3-Methyl-4-octanolid erhalten wird, 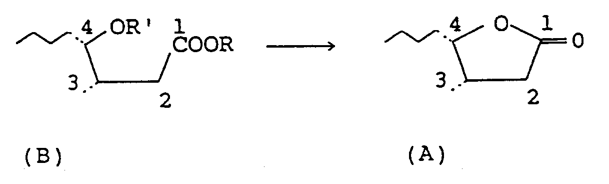 (wobei R und R' die gleichen Bedeutungen wie oben angegeben aufweisen).

3. Verfahren zur Herstellung von cis-Whiskey-lacton nach Anspruch 1, umfassend die nachfolgenden Schritte:
(a) Hydrolysieren des Lactonanteils von (3R,4S)-3-Methyl-4-octanolid der allgemeinen Formel (D') und Anlagerung einer Alkylgruppe an den hydrolysierten Teil, wodurch die in der allgemeinen Formel (C') gezeigte 3,4-trans-Verbindung erhalten wird, 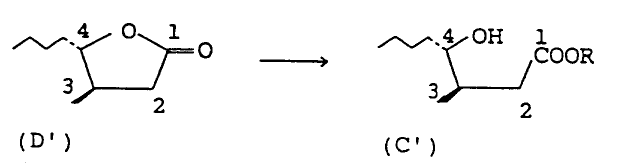 (wobei R eine allgemeine Alkylgruppe und R' eine allgemeine Acylgruppe repräsentieren),
(b) Umsetzen der im Schritt (a) erhaltenen 3,4-trans-Verbindung (C') mit einer Acylverbindung in Gegenwart von Triphenylphosphin und Azodicarboxylat, wodurch die in der allgemeinen Formel (B') gezeigte 3,4-cis-Verbindung erhalten wird 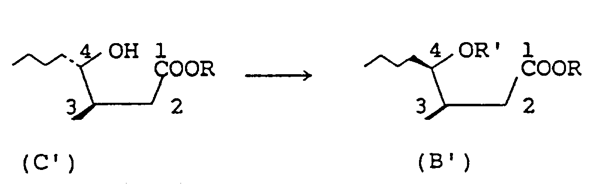 (worin R und R' die gleichen Bedeutungen wie oben angegeben aufweisen), und
(c) Hydrolysieren und Lactonisieren der im Schritt (b) erhaltenen 3,4-cis-Verbindung (B'), wodurch das in der allgemeinen Formel (A') gezeigte (3R,4R)-3-Methyl-4-octanolid erhalten wird 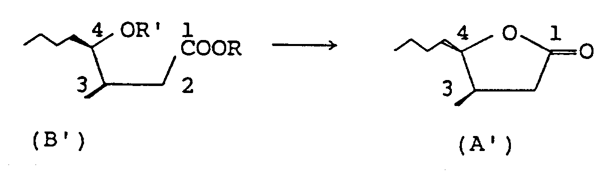 (wobei R und R' die gleichen Bedeutungen wie oben angegeben aufweisen).

## Revendications

1. Procédé de fabrication de la cis-lactone de whisky comprenant :
(a) une étape d'hydrolyse de la portion lactone de la 3,4-trans-lactone du whisky de formule générale (IV) et d'addition d'un groupe alkyle sur la portion hydrolysée, pour obtenir ainsi un composé 3,4-trans représenté par la formule générale (III), 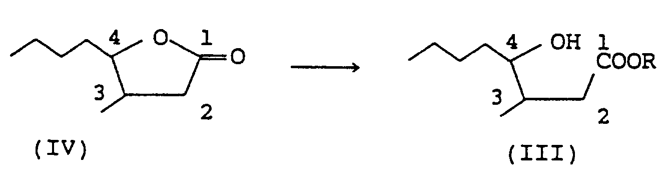 (où R représente un groupe alkyle général et R' représente un groupe acyle général)
(b) une étape de réaction du composé 3,4-trans (III) obtenu dans l'étape (a) avec un composé acylé en présence de triphénylphosphine et d'un azodicarboxylate, pour obtenir ainsi un composé 3,4-cis représenté par la formule (II), 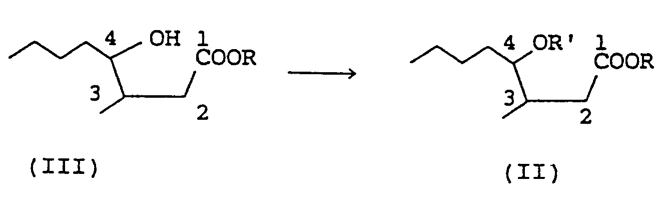 (où R et R' sont définis comme ci-dessus) et
(c) une étape d'hydrolyse et de lactonisation du composé 3,4-cis (II) obtenu dans l'étape (b), pour obtenir ainsi la 3,4-cis-lactone du whisky représentée par la formule générale (I) 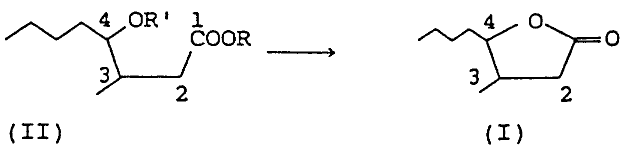 (où R et R' sont définis comme ci-dessus).

2. Procédé de fabrication de la cis-lactone du whisky selon la revendication 1, comprenant
(a) une étape d'hydrolyse de la portion lactone du (3S,4R)-3-méthyl-4-octanolide représenté par la formule générale (D) et d'addition d'un groupe alkyle sur la portion hydrolysée, pour obtenir ainsi le composé 3,4-trans représenté par la formule générale (C), 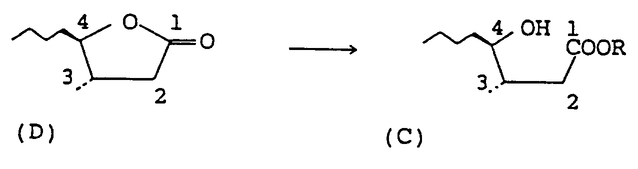 (où R représente un groupe alkyle général et R' représente un groupe acyle général)
(b) une étape de réaction du composé 3,4-trans (C) obtenu dans l'étape (a) avec un composé acylé en présence de triphénylphosphine et d'un azodicarboxylate, pour obtenir ainsi le composé 3,4-cis représenté par la formule générale (B), 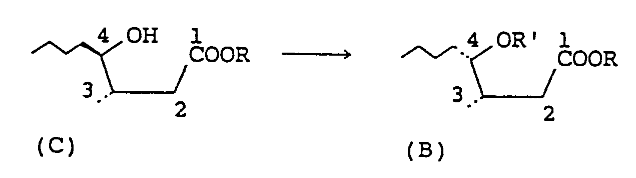 (où R et R' sont définis comme ci-dessus) et
(c) une étape d'hydrolyse et de lactonisation du composé 3,4-cis (B) obtenu dans l'étape (b), pour obtenir ainsi le (3S,4S)-3-méthyl-4-octanolide représenté par la formule générale (A) 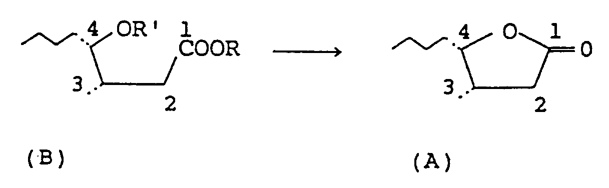 (où R et R' sont définis comme ci-dessus).

3. Procédé de fabrication de la cis-lactone du whisky selon la revendication 1, comprenant
(a) une étape d'hydrolyse de la portion lactone du (3R,4S)-3-méthyl-4-octanolide représenté par la formule générale (D') et d'addition d'un groupe alkyle à la portion hydrolysée, pour obtenir ainsi le composé 3,4-trans représenté par la formule générale (C'), 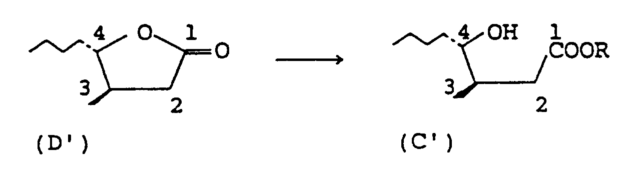 (où R représente un groupe alkyle général et R' représente un groupe acyle général)
(b) une étape de réaction du composé 3,4-trans (C') obtenu dans l'étape (a) avec un composé acylé en présence de triphénylphosphine et d'un azodicarboxylate, pour obtenir ainsi le composé 3,4-cis représenté par la formule générale (B'), 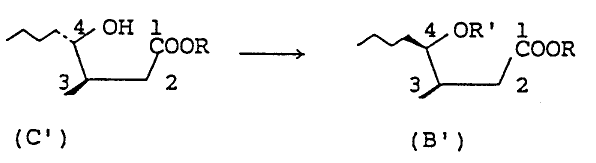 (où R et R' sont définis comme ci-dessus) et
(c) une étape d'hydrolyse et de lactonisation du composé 3,4-cis (B') obtenu dans l'étape (b), pour obtenir ainsi le (3R,4R)-3-méthyl-4-octanolide représenté par la formule générale (A') 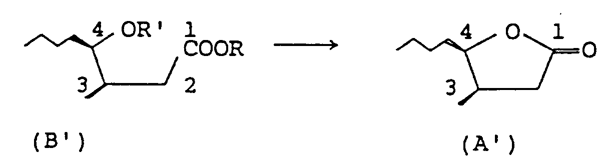 (où R et R' sont définis comme ci-dessus).
